# EUROPEAN PATENT APPLICATION

(11) **EP 4 458 301 A1**
(43) Date of publication of application: **06.11.2024**
(21) Application number: 22914683.2
(22) Date of filing: 26.12.2022
(51) Int. Cl.: A61B 18/14, A61B 18/12

(54) **NERVE ABLATION APPARATUS**

(30) Priority: 31.12.2021 CN 202111677021
(71) Applicant: Shenzhen Lifetech Respiration Scientific Co., Ltd., Shenzhen, Guangdong 518057 (CN)
(72) Inventor: HU, Longhu, Shenzhen, Guangdong 518057 (CN); SHI, Yue, Shenzhen, Guangdong 518057 (CN); LI, Anning, Shenzhen, Guangdong 518057 (CN)
(74) Representative: Prinz & Partner mbB
(86) International application number: PCT/CN2022/141979
(87) International publication number: WO 2023/125429

(57) **Abstract**

A nerve ablation apparatus is provided, including supporting bodies (110, 210, 310, 410) and mounting members (120, 220, 420); the mounting members (120, 220, 420) are provided along the circumferences of the supporting bodies (110, 210, 310, 410) and of mesh structures; the mounting members (120, 220, 420) include first regions (121, 222) and second regions (122, 223) provided along the circumferences of the supporting bodies (110, 210, 310, 410) and include the mesh structures formed by a plurality of mesh wires; in the first regions (121, 222), the plurality of mesh wires intersect and at least a portion of the mesh wires are fixedly connected at intersections. Thus, when the supporting bodies (110, 210, 310, 410) are released in an airway (810), the supporting bodies (110, 210, 310, 410) push the mounting members (120, 220, 420) to attach to an inner wall of the airway (810); when the first regions (121, 222) and the second regions (122, 223) are compressed by the airway (810), reduction amounts of areas of meshes on the first regions (121, 222) are small, and when the first regions (121, 222) and the second regions (122, 223) are energized, ablation energy emitted by the first regions (121, 222) is less than ablation energy emitted by the second regions (122, 223), thereby reducing the burn of an esophagus (820) by the mounting members (120, 220, 420).

## Description

### Technical Field

The present invention relates to the field of medical devices, and particularly to a nerve ablation apparatus.

### Background Art

This section merely provides background information related to the present disclosure and is not necessarily related art.

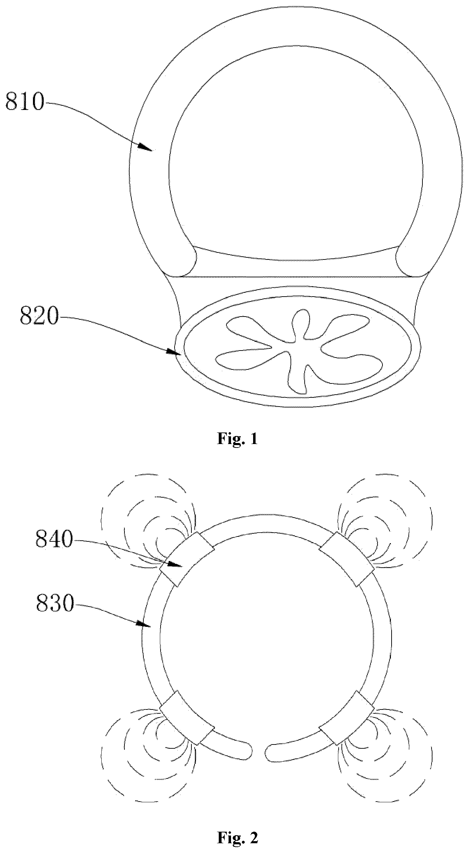

As shown in Figs. 1 and 2, a cross-section of an airway 810 has an approximately D-shaped profile, and an esophagus 820 generally attaches to a relatively flat side of the airway 810. In the related art, a plurality of electrodes 840 on a nerve ablation apparatus are generally circumferentially distributed on an electrode supporting structure 830. After the electrodes 840 attach to a site to be ablated, the electrodes 840 output radio frequency (RF) energy to destroy nerves on tissues with the electrodes 840 themselves as a center to achieve the effect of blocking nerves. However, in order to ablate a region away from the electrodes 840, the electrodes 840 need to output sufficient RF energy to achieve the ablation of the region away from the electrodes 840, which results in high RF energy in a central region, thereby easily causing burns to the esophagus 820 attached to the airway 810.

### Summary of the Invention

Based on the above, it is necessary to provide a nerve ablation apparatus, including a supporting body and a mounting member, where the mounting member is provided along a circumference of the supporting body and of a mesh structure; the mounting member includes a first region and a second region provided along the circumference of the supporting body and includes the mesh structure formed by a plurality of mesh wires; in the first region, the plurality of mesh wires intersect and at least a portion of the mesh wires are fixedly connected at intersections.

Optionally, the mesh wires include woven wires, and the mesh structure is formed by weaving the woven wires.

Optionally, the amount of increase of the mesh density in the first region is less than the amount of increase of the mesh density in the second region when the mounting member is subjected to pressure towards an inner side of the supporting body.

Optionally, the nerve ablation apparatus further includes a buffer structure; the buffer structure is provided at intersection points of the first region and the second region and is configured to block or weaken the transmission of force between the first region and the second region.

Optionally, the buffer structure includes a reinforcing member; the reinforcing member is connected to the supporting body and the mesh wires and extends from the supporting body to the intersection points of the first region and the second region.

Optionally, the mesh wire is made of conductive material to make the mounting member form mesh electrodes.

Optionally, the stiffness of one end of the supporting body close to the mounting member is less than the stiffness of the other end of the supporting body away from the mounting member.

Optionally, the supporting body has a mesh structure, and the area of the smallest mesh of an end portion of the supporting body close to the mounting member is larger than the area of the smallest mesh of an end portion of the supporting body away from the mounting member.

Optionally, the cross-sectional area of the supporting body gradually decreases from one end close to the mounting member to one end away from the mounting member.

Optionally, at least a portion of the mounting member is bent towards a direction close to the center of the supporting body, and the side wall of the mounting member includes an arcuate curved surface.

Optionally, the supporting body includes a disc-shaped woven body formed by weaving at least one woven wire, and the mounting member is provided on a circumferential side wall of the disc-shaped woven body.

Optionally, the nerve ablation apparatus further includes a cooling balloon; the cooling balloon is inserted into the supporting body, and after the cooling balloon is inflated, the side wall of the cooling balloon attaches to a side wall of the supporting body.

Optionally, heat conduction holes are arranged on the side wall of the supporting body, and after the cooling balloon is inflated, the cooling balloon covers the heat conduction holes.

Compared with the related art, the beneficial effects of the nerve ablation apparatus of the present invention are as follows.

When the supporting body is released in an airway, it pushes the mounting member to attach to an inner wall of the airway. The first region attaches to a side wall of the airway close to an esophagus, and the second region attaches to a side wall of the airway away from the esophagus. When the first region and the second region are compressed by the airway, the reduction amount of the area of the mesh on the first region is small, and the reduction amount of the area of the mesh on the second region is large, namely, conductive wires on the first region are sparse, and conductive wires on the second region are dense. When the first region and the second region are energized, ablation energy emitted by the first region is less than ablation energy emitted by the second region, thereby reducing the burn of the esophagus by the mounting member.

### Brief Description of the Drawings

Fig. 1 is a schematic structural diagram of a cross-section of an airway in the related art;
Fig. 2 is a schematic diagram of an electrode supporting structure in the related art;
Fig. 3 is a schematic structural diagram of an ablation apparatus according to embodiment 1 of the present invention;
Fig. 4 is a schematic structural diagram of a mounting member according to embodiment 1 of the present invention;
Fig. 5 is a schematic structural diagram of an electrode emitting an ablation energy field according to embodiment 1 of the present invention;
Fig. 6 is a schematic structural diagram of a fixed connection point according to embodiment 1 of the present invention;
Fig. 7 is another schematic structural diagram of a fixed connection point according to embodiment 1 of the present invention;
Fig. 8 is a schematic structural diagram of a mounting member being compressed by an airway according to embodiment 1 of the present invention;
Fig. 9 is a schematic structural diagram of the connection of a mounting member and a supporting body according to embodiment 1 of the present invention;
Fig. 10 is a schematic structural diagram showing a gap between an electrode supporting body and an inner wall of an airway in the related art;
Fig. 11 is a schematic structural diagram of the connection of a buffer structure according to embodiment 1 of the present invention;
Fig. 12 is an enlarged view of a structure at C of FIG. 11 according to the present invention;
Fig. 13 is an expanded schematic structural diagram of a mounting member according to embodiment 1 of the present invention;
Fig. 14 is a schematic structural diagram showing the mounting of electrodes on a mounting member according to embodiment 1 of the present invention;
Fig. 15 is a schematic structural diagram of an ablation apparatus according to embodiment 2 of the present invention;
Fig. 16 is a schematic structural diagram of an ablation apparatus according to embodiment 3 of the present invention;
Fig. 17 is a schematic structural diagram of a cooling balloon according to embodiment 4 of the present invention; and
Fig. 18 is a schematic structural diagram of a cooling conduit according to embodiment 4 of the present invention.

### Detailed Description of the Invention

In order to make the above-mentioned objects, features, and advantages of the present invention more obvious and understandable, the specific implementations of the present invention are described in detail below in conjunction with the accompanying drawings. Many specific details are set forth in the following description to facilitate a full understanding of the present invention. However, the present invention can be implemented in many other ways different from those described herein, and a person skilled in the art may make similar improvements without contravening the connotation of the present invention, and therefore the present invention is not subject to the specific implementations disclosed below.

Unless otherwise defined, all technical and scientific terms used herein have the same meanings as generally understood by a person skilled in the art of the present invention. The terms used in the description of the present invention herein are for the purpose of describing specific embodiments only and are not intended to limit the present invention.

### Embodiment 1

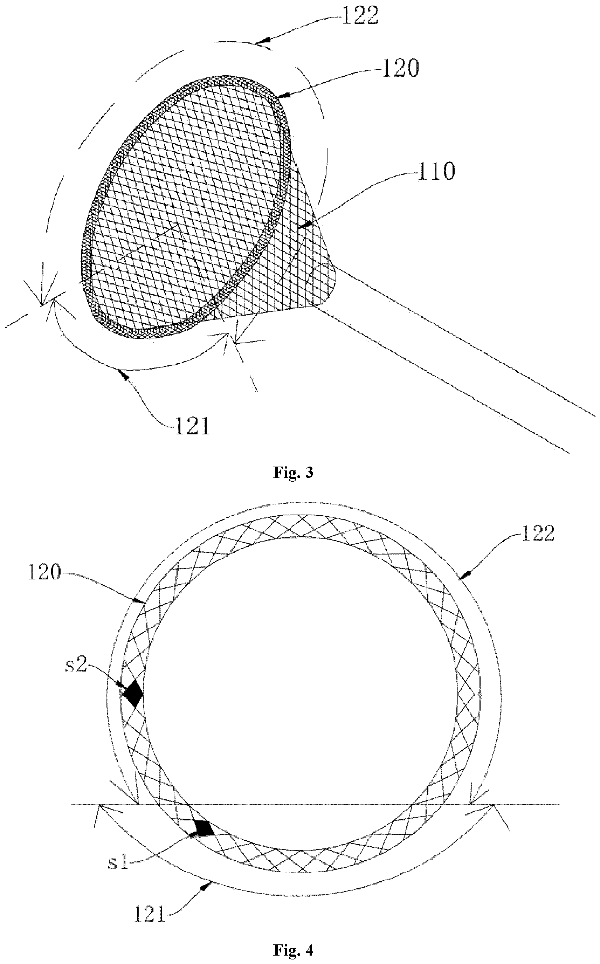

An embodiment of the present invention provides an ablation apparatus, as shown in Figs. 3 to 9, including a supporting body 110 and a mounting member 120. The mounting member 120 is provided along a circumference of the supporting body 110 and of a mesh structure. The mounting member 120 includes a first region 121 and a second region 122, and when the mounting member 120 is compressed by an airway 810, the amount of the mesh density in the first region 121 that increases is less than the amount of the mesh density in the second region 122 that increases.

The ablation apparatus is configured to output ablation energy at the tissue to be ablated in human body, and the ablation energy ablates nerves in the tissue, thereby achieving the effect of treating a lesion site of human body. The ablation apparatus can be used for ablation of airway, heart, aorta, stomach, etc. For example, in this embodiment, the ablation apparatus is delivered into the airway 810 through a delivery apparatus, and then the ablation apparatus ablates nerves in the tissue of the airway 810, thereby expanding the airway 810 and achieving treatment of a pulmonary disease.

The supporting body 110 includes a loading state and an expanded state. When the supporting body 110 is located in the delivery apparatus, the supporting body 110 is in the loading state. At this time, the supporting body 110 is compressed in a sheath of the delivery apparatus to facilitate delivery of the supporting body 110. When the supporting body 110 is released in the airway 810, the supporting body 110 self- expands, expanding the mounting member 120 to attach to an inner wall of the airway 810.

The supporting body 110 can be in the shape of a tube, a rod, a disc, or a square and can be formed by cutting memory metal material or by weaving filamentary material. The supporting body 110 has a certain elasticity, and when the mounting member 120 is compressed by the airway 810, the supporting body 110 provides an elastic supporting force for the mounting member 120 to ensure a contact area between the mounting member 120 and the inner wall of the airway 810.

The mounting member 120 can be formed by weaving, by cutting metal material, or be a mesh structure made of conductive material. The mounting member 120 is provided around the supporting body 110 and can be wound on the supporting body 110, welded or integrally connected to the supporting body 110. An outer side wall of the mounting member 120 has a mesh structure and attaches to the inner wall of the airway 810. In other embodiments, one axial end of the supporting body 110 is bent radially outward and forms the mounting member 120 by heat setting. The mounting member 120 is configured to mount electrodes 840, and the electrodes 840 form ablation energy fields after being mounted on the mounting member 120 to achieve ablation of the tissue of the airway 810.

In this embodiment, the mounting member 120 and the supporting body 110 are both formed by weaving conductive wires, and the conductive wires on the mounting member 120 are exposed to form mesh electrodes 840. An outer side wall of the conductive wire on the supporting body 110 is covered with an insulating layer. After energizing the conductive wire, the mounting member 120 forms the mesh electrodes 840 to output RF energy to destroy nerves in the tissue of the airway 810.

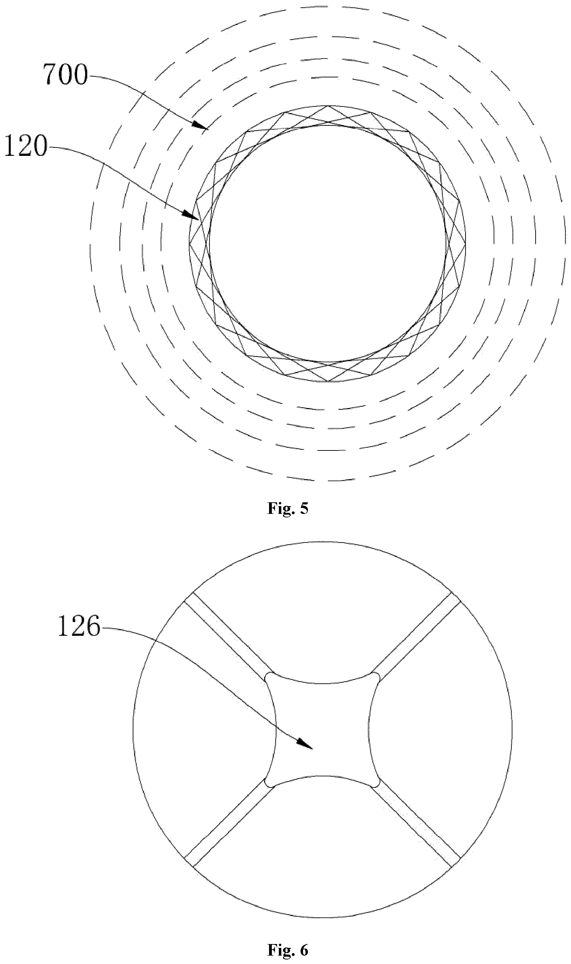

The conductive wire can be a nickel-titanium alloy wire, a stainless-steel wire, or any other wire that can be conductive. One end of the conductive wire is connected to an RF (Radio Frequency) energy meter. The RF energy meter provides electric energy for the conductive wire, and the electric energy passes through the supporting body 110 and is transmitted to the mounting member 120 to form an ablation energy field 700. As shown in Fig. 5, the ablation energy field 700 takes the supporting body 110 as a center and then spreads out radially layer by layer from a position where the mounting member 120 attaches to the airway 810.

As shown in Fig. 5, the mounting member 120 can be regarded as a point electrode so that a continuous annular ablation energy field 700 can be formed with the mounting member 120 as the center and the position where the mounting member 120 attaches to the airway 810 as a starting point. Compared with an ablation mode in which a plurality of point electrodes 840 generate a plurality of overlapping annular energy fields, the energy of the mesh electrode is more uniform, and outputted ablation energy is more easily controlled, reducing the probability of the ablation apparatus causing burns to the airway 810.

Further, in order to reduce the damage to the esophagus 820 during the ablation, as shown in FIG. 4, the mounting member 120 includes the first region 121 and the second region 122 provided along the circumference of the supporting body 110, and when the mounting member 120 is compressed by the airway 810, the amount of the mesh density in the first region 121 that increases is less than the amount of the mesh density in the second region 122 that increases.

It should be noted that the mesh density in the embodiment refers to a number of meshes per unit area, and the amount of the mesh density that increases refers to a number of meshes per unit area when the supporting body 110 is not compressed minus a number of meshes per unit area when the supporting body 110 is compressed. The first region 121 and the second region 122 are provided along the circumference of the mounting member 120, and the ratio of circumferential lengths of the first region 121 and the second region 122 is: 0.3-1. Specifically, the ratio of the circumferential lengths of the first region 121 and the second region 122 is 0.3, 0.5, 0.6, 0.8, or 1.

It is worth explaining that the measurement of the mesh density may be achieved by electron microscope photogrammetry. For example, in one embodiment, a transparent first fixture in the shape of a rectangular is provided. A first lumen structure corresponding to a profile of the mounting member 120 is arranged on the first fixture, and a plurality of measurement square grids having the same area are provided on an outer side wall of the first fixture. After the mounting member 120 is released in the first lumen structure, the side wall of the mounting member 120 attaches to an inner wall of the first lumen structure without an interaction force. An electron microscope is used to magnify the first fixture by a certain multiple, and a mesh number t1 in a single measurement square grid is calculated by a mesh method. A second fixture corresponding to a cross-section of the airway is provided. A second lumen structure corresponding to a profile of the airway is arranged on the second fixture, and a plurality of measurement squares having the same area are provided on an outer side wall of the second fixture. The mounting member 120 is released in the second lumen structure and attaches to an inner wall of the second lumen structure. The electron microscope is used to magnify the second fixture by a certain multiple, and a mesh number t2 in a single measurement square is calculated by the mesh method. An increasing amount of an area of the mesh is t2-t1.

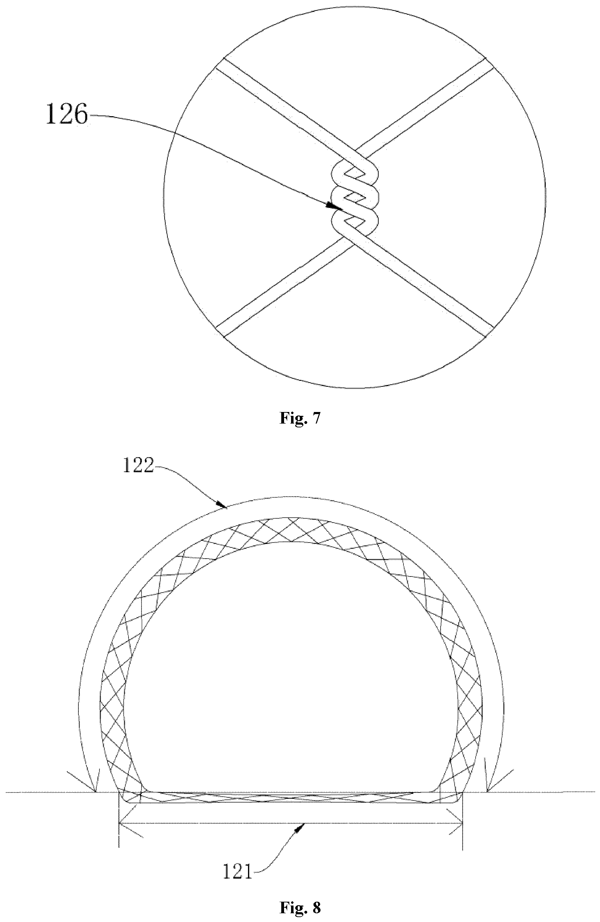

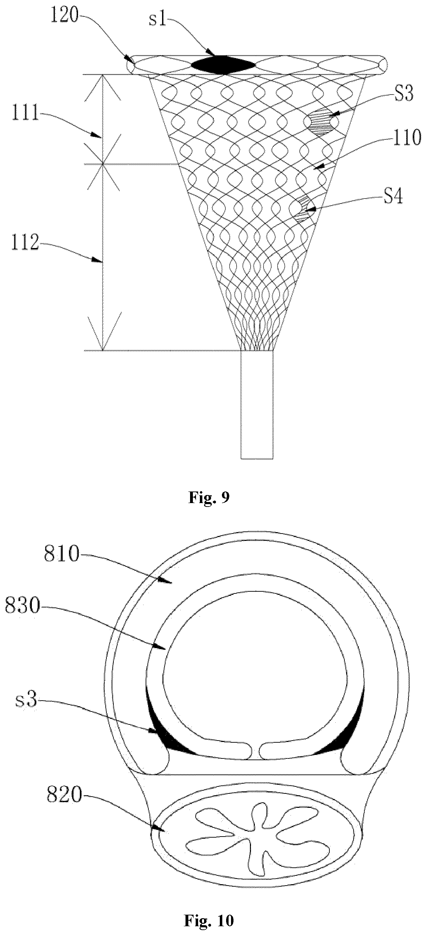

In this embodiment, the mounting member 120 includes a plurality of mesh wires, which form a mesh structure. The mesh wires are formed by weaving woven wires, which can be conductive wires, nickel-titanium alloy wires, or stainless-steel wires. For example, as shown in Figs. 6 and Figs. 7, at the first region 121, the conductive wires intersect and are fixedly connected, and at the second region 122, the conductive wires intersect and are movably connected. For example, at the first region 121, the conductive wires intersect to form a grid or mesh, and two intersecting conductive wires are welded or wound at a fixed connection point 126. Thus, as shown in Figs. 8 and 9, at the first region 121, when the conductive wires are compressed, the two intersecting conductive wires are already fixed so that a displacement of two adjacent conductive wires moving towards each other is small or maintained constant. Therefore, an area s1 of the mesh on the first region 121 is constant, or a reduction amount of the area s1 of the mesh is small, and the amount of increase for the mesh density in the first region 121 is small. At the second region 122, the two intersecting conductive wires attach to each other and may move away from each other or move towards each other. Thus, at the second region 122, when the conductive wires are compressed, the displacement of the two intersecting conductive wires moving towards each other is large. Therefore, the reduction amount of an area s2 of the mesh in the second region 122 is large, namely, the increasing amount of the mesh density in the second region 122 is large.

In another embodiment, the mounting member 120 includes mesh wires formed by cutting metal material. For example, in one embodiment, the first region 121 includes arcuate mesh wires formed by cutting metal material, and the second region 122 has an arcuate mesh structure formed by weaving the conductive wires. The conductive wires on the second region 122 intersect and are movably connected, and two longitudinal ends of the first region 121 and the second region 122 are attached. Thus, when the first region 121 and the second region 122 are compressed by the airway 810, the first region 121 is formed by cutting metal material so that the area s1 of the mesh can be maintained unchanged when the first region 121 is compressed. The second region 122 is formed by weaving the conductive wires, and the conductive wires on the second region 122 intersect and are movably connected so that the reduction amount of the area s2 of the mesh in the second region 122 is large when the second region 122 is compressed by the airway 810, thereby realizing the amount of increase for the mesh density in the first region 121 being less than the amount of increase for the mesh density in the second region 122.

The advantage of this arrangement is that when the supporting body 110 is released in the airway 810, it pushes the mounting member 120 to attach to the inner wall of the airway 810. The first region 121 attaches to a side wall of the airway 810 close to the esophagus 820, and the second region 122 attaches to a side wall of the airway 810 away from the esophagus 820. When the first region 121 and the second region 122 are compressed by the airway 810, the reduction amount of the area of the mesh on the first region 121 is small, and the reduction amount of the area of the mesh on the second region 122 is large, namely, the conductive wires on the first region 121 are sparse, and the conductive wires on the second region 122 are dense. When the first region 121 and the second region 122 are energized, ablation energy emitted by the first region 121 is less than ablation energy emitted by the second region 122, thereby reducing the burn of the esophagus 820 by the mounting member 120.

Further, in order to reduce the interference of the supporting body 110 with the deformation of the first region 121, the stiffness of one end of the supporting body close to the mounting member 120 is less than the stiffness of one end of the supporting body away from the mounting member 120.

In this embodiment, as shown in Fig. 9, the supporting body 110 has a mesh structure and includes a first end portion 111 and a second end portion 112. The first end portion 111 is connected to the mounting member 120, and the area of the smallest mesh on the first end portion 111 is larger than the area of the smallest mesh on the second end portion 112.

It should be noted that the first end portion 111 refers to one end covered by a radial projection of the mounting member 120 on the supporting body 110, and the second end portion 112 refers to the other end of the supporting body 110 away from the mounting member 120. The smallest mesh on the first end portion 111 refers to an area S3 of the smallest mesh formed by the conductive wires intersecting on the first end portion 111, and the smallest mesh on the second end portion 112 refers to an area S4 of the smallest mesh formed by the conductive wires intersecting on the second end portion 112. The area of the smallest mesh on the first end portion 111 is greater than the area of the smallest mesh on the second end portion 112, namely, the area of any one of the meshes on the first end portion 111 is greater than the area of the mesh on the second end portion 112.

The area of the mesh on the first end portion 111 is large, namely, the density of the woven wires on the first end portion 111 is low. At this time, the deformation performance of the first end portion 111 is greater than the deformation performance of the second end portion 112. When the mounting member 120 is compressed by the airway 810, the first end portion 111 can be followed the second end portion 112 to deform, avoiding the interference of the supporting body 110 on the deformation of the mounting member 120, making the mounting member 120 more easily adapt to a D-shaped profile of the airway 810, and increasing the adherence of the mounting member 120. The area of the mesh on the second end portion 112 is smaller, namely, the density of the conductive wires on the second end portion 112 is low. At this time, the deformation resistance of the second end portion 112 is greater than the deformation resistance of the first end portion 111 so that the second end portion 112 can provide a supporting force for the first end portion 111 and increase the stability of the delivery and release of the supporting body 110.

In another embodiment, the diameter of the woven wire at the first end portion 111 is smaller than the diameter of the woven wire at the second end portion 112. Since the stiffness of the woven wire with a larger diameter is greater than the stiffness of the woven wire with a smaller diameter, the stiffness at the first end portion 111 is less than the stiffness at the second end portion 112, thereby achieving the arrangement that the stiffness of one end of the supporting body close to the mounting member 120 is less than the stiffness of one end of the supporting body away from the mounting member 120.

In other embodiments, the woven wires at the first end portion 111 intersect and are movably connected, and the woven wires at the second end portion 112 intersect and are fixedly connected. Since the deformation resistance of the fixedly connected woven wires is greater than the deformation resistance of the movably connected woven wires, the stiffness at the first end portion 111 is less than the stiffness at the second end portion 112, thereby achieving the arrangement that the stiffness of one end of the supporting body close to the mounting member 120 is less than the stiffness of one end of the supporting body away from the mounting member 120.

It is worth explaining that, since the stress of the supporting body 110 in the loading state is large, when the supporting body 110 is released in the airway 810, the supporting body 110 tends to be instantaneously expanded and destabilized. At this time, since the second end portion 112 has a higher stiffness, it has a stronger supporting force so that the second end portion 112 can slow down the deformation rate when the first end portion 111 is released, thereby making the first end portion 111 more stable when released.

Further, in order to reduce the difficulty of loading the ablation apparatus into the delivery apparatus, as shown in Fig. 9, a cross-sectional area of the supporting body 110 gradually decreases from the first end portion 111 to the second end portion 112. In this embodiment, the cross-sectional area of the supporting body 110 refers to the cross-sectional area of the supporting body 110 on a radial plane, and it gradually decreases from the first end portion 111 to the second end portion 112 so that the supporting body 110 defines a truncated cone-shaped structure as a whole.

Since the conductive wires of the second end portion 112 are dense, it is more difficult for the supporting body 110 to enter the sheath of the delivery apparatus. The cross-sectional area of the supporting body 110 gradually decreases from the first end portion 111 to the second end portion 112 so that the cross-sectional area of the supporting body 110 at the second end portion 112 is small, thereby reducing the difficulty of the second end portion 112 entering the sheath.

It is worth explaining that, as shown in Fig. 10, with regard to an annular electrode supporting structure 830, when the electrode supporting structure 830 is released in the airway 810, the pressure of a smooth muscle 812 of the airway 810 on the electrode supporting structure 830 is greater than the pressure of a C-shaped region of the airway 810 on the electrode supporting structure 830, and the deformation of one side of the electrode supporting structure 830 close to the smooth muscle 812 is greater than the deformation of one side of the electrode supporting structure 830 close to the C-shaped region. At this time, the deformation of the side of the electrode supporting structure 830 close to the smooth muscle 812 causes excessive deformation of the side of the electrode supporting structure 830 close to the C-shaped region so that a gap s3 is formed between the electrode supporting structure 830 and the inner wall of the airway 810 at a transition region where the C-shaped region connects to the smooth muscle 812, resulting in difficulty in ablating the airway at the gap s3.

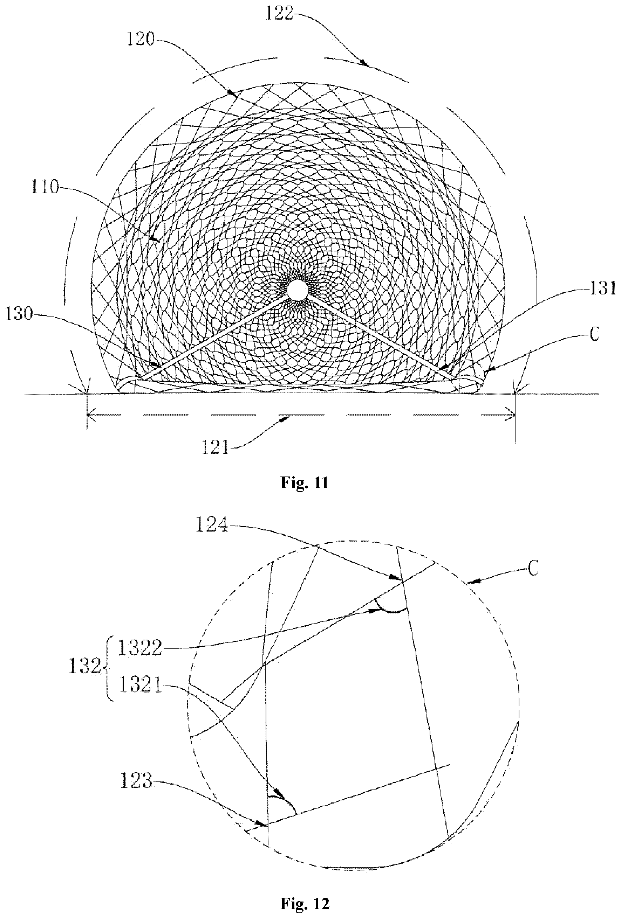

Further, in order to facilitate adherence of the mounting member 120 to the D-shaped profile of the airway 810, as shown in Fig. 11, the ablation apparatus further includes a buffer structure 130. The buffer structure 130 is connected to the mounting member 120 at connection points of the first region 121 and the second region 122.

In one embodiment, as shown in Fig. 11, the buffer structure 130 includes reinforcing rods 131. The reinforcing rod 131 is welded or inserted on the supporting body 110 and extends to the mounting member 120 along the side wall of the supporting body 110. The reinforcing rod 131 forms a deformation-resistant support at the connection point of the first region 121 and the second region 122 and reduces or blocks the transmission of force from the first region 121 to the second region 122. Therefore, when the first region 121 is compressed by the smooth muscle 812, the traction of the first region 121 on the second region 122 is blocked or weakened by the reinforcing rod 131 so that the second region 122 is not affected by the deformation of the first region 121, and the second region 122 attaches to the C-shaped profile 811, improving the adherence of the mounting member 120.

In another embodiment, as shown in Fig. 12, the buffer structure 130 includes reinforcing ribs 132. In a region where the first region 121 and the second region 122 are connected, the reinforcing rib 132 is located in the mesh and connected to the woven wires of the mesh intersecting at two circumferential ends of the mounting member 120. The mesh in the region where the first region 121 and the second region 122 are connected includes a first intersection point 123 and a second intersection point 124 where the woven wires intersect, and the first intersection point 123 and the second intersection point 124 are provided along the circumference of the mounting member 120. The reinforcing rib 132 includes a first reinforcing rib 1321 and a second reinforcing rib 1322. Two ends of the first reinforcing rib 1321 are connected to two woven wires on the first intersection point 123, and two ends of the second reinforcing rib 1322 are connected to two woven wires on the second intersection point 124. The first reinforcing rib 1321 and the second reinforcing rib 1322 are both in an arcuate structure, and arcuate convex surfaces of the first reinforcing rib 1321 and the second reinforcing rib 1322 are provided opposite to each other. In this way, by providing the reinforcing rib 132, a deformation-resistant support may be formed at a position where the first region 121 and the second region 122 are connected, blocking or weakening the transmission of force from the first region 121 to the second region 122 so that the second region 122 is not affected by the deformation of the first region 121, and the second region 122 may attach to the C-shaped profile 811, improving the adherence of the mounting member 120.

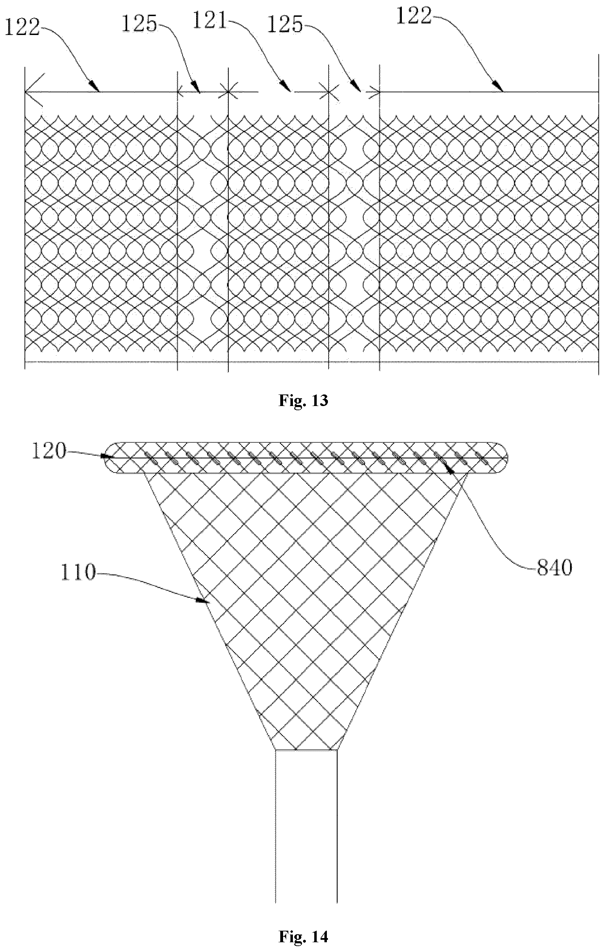

Further, as shown in Fig. 13, the mounting member 120 further includes third regions 125. The third regions 125 are located between the first region 121 and the second region 122 and form free sections to slow down the transmission of force from the first region 121 to the second region 122. It will be appreciated that forming the third region 125 as a free section is one embodiment of the buffer structure 130.

In this embodiment, the free section refers to an unconstrained section, namely, at the third region 125, where the transmission effect of force is low. Specifically, the woven wires at the third region 125 intersect and are movably connected, and the area of the mesh formed by the intersection of the woven wires is much larger than the area of any mesh of the first region 121 and/or the second region 122. Thus, at the third region 125, the density of the woven wires is low, and the transmission effect of force from the first region 121 to the second region 122 is weakened so that the second region 122 is not affected by the deformation of the first region 121, and the second region 122 attaches to the C-shaped profile 811, improving the adherence of the mounting member 120.

Further, the ratio of the circumferential lengths of the second region 122 and the first region 121 is 1.1-4. Specifically, the ratio of the circumferential lengths of the second region 122 and the first region 121 can be 1.1, 1.5, 2, 3, or 4. Thus, by setting the ratio of the circumferential lengths of the second region 122 and the first region 121, a transition region of the first region 121 and the second region 122 corresponds to a transition region of the C-shaped profile 811 of the airway 810 and the smooth muscle 812, thereby improving the adaptability to the airway 810.

Further, in order to decrease a frictional force between the mounting member and the inner wall of the airway, as shown in Fig. 14, the mounting member 120 has an arcuate structure, and a convex surface of the arcuate structure is provided toward the side away from the supporting body 110.

In this embodiment, the mounting member 120 is formed by bending one axial end of the supporting body 110 to the outside of the supporting body 110, then bending to the inside of the supporting body 110, and finally by heat setting. During the release of the supporting body 110 in the airway 810, the mounting member 120 is likely to not attach to a target region. At this time, it is necessary for an operator to move the mounting member 120 to make the mounting member 120 attach to the target region. During the movement of the mounting member 120, a frictional force between the end portion of the mounting member 120 and the airway 810 is large, which may easily rub the airway 810 or cause discomfort to a patient.

Thus, the arcuate structure of the mounting member 120 can attach to the inner wall of the airway 810, the contact area between the arcuate structure and the inner wall of the airway 810 is increased, and the frictional force between the mounting member 120 and the inner wall of the airway 810 is reduced. By providing the convex surface of the arcuate structure toward the side away from the supporting body 110, the end portion of the mounting member 120 is provided toward the inner side of the supporting body 110, and during the movement of the mounting member 120, the friction between the end portion of the mounting member 120 and the airway 810 and discomfort to the patient may be prevented.

In other embodiments, as shown in Fig. 14, the ablation apparatus includes electrodes 840, and the electrodes 840 are provided on the mounting member 120. The electrode 840 may be an elongated electrode 840 or a point electrode 840 and is provided on the mounting member 120 along the circumference of the supporting body 110. The electrode 840 may be welded or wound on the mounting member 120 and is connected to the RF energy meter. The RF energy meter energizes the electrode 840 so that the electrode 840 emits the ablation energy field 700.

The advantage of this arrangement is that when the mounting member 120 is compressed by the airway 810, the woven wires on the first region 121 intersect and are fixedly connected, and the woven wires on the second region 122 intersect and are movably connected so that the reduction amount of the mesh on the first region 121 is less than the reduction amount of the mesh on the second region 122, namely, the amount of movement of different electrodes 840 on the first region 121 moving towards each other is smaller or unchanged, and the amount of movement of different electrodes 840 on the second region 122 moving towards each other is greater. Furthermore, the degree of overlap of the energy fields of the electrodes 840 on the first region 121 is lower than the degree of overlap of the energy fields of the electrodes 840 on the second region 122, namely, the ablation energy emitted by the first region 121 is less than the ablation energy emitted by the second region 122, reducing the burn of the esophagus 820 by the mounting member 120.

### Embodiment 2

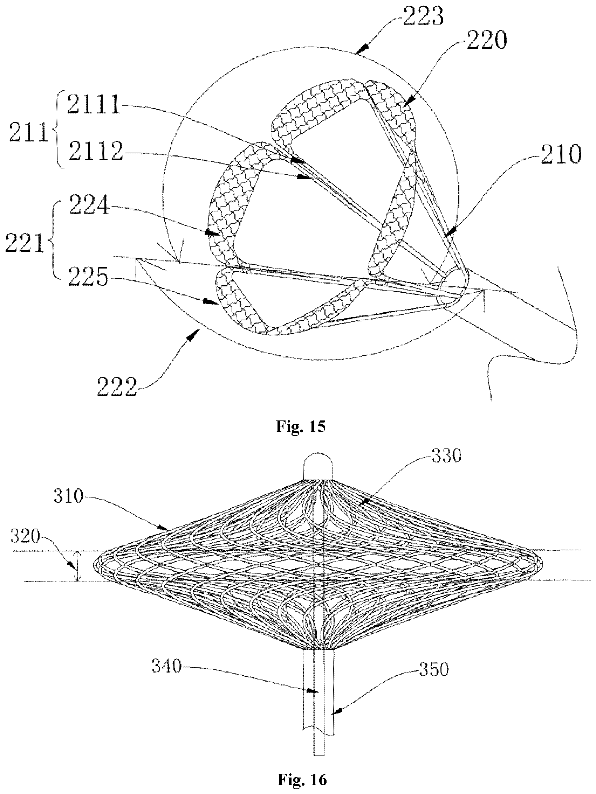

The embodiment differs from embodiment 1 in that, as shown in Fig. 15, the mounting member 220 includes a plurality of ablation units 221 provided along the circumference of the supporting body 210, and two circumferentially adjacent ablation units 221 are provided at spaced intervals. The ablation units 221 are provided on the supporting body 210 and are located on the first region 222 and/or the second region 223.

In this embodiment, the mounting member 220 forms a plurality of ablation units 221 during weaving. The ablation units 221 are of a mesh structure with an arcuate edge profile, and two adjacent ablation units 221 have a spacing distance at a portion away from the supporting body 210 to allow a deformation space of the ablation units 221. The ablation units 221 may be provided on the first region 222 or the second region 223 at the same time, or only on the first region 222.

For example, in one embodiment, the ablation unit 221 includes a first ablation unit 224 and a second ablation unit 225. The first ablation unit 224 is located on the first region 222, and the second ablation unit 225 is located on the second region 223. The conductive wires on the first ablation unit 224 intersect and are fixedly connected, and the conductive wires on the second ablation unit 225 intersect and are movably connected. Two circumferentially adjacent ablation units 221 have a certain spacing distance.

In this way, when the mounting member 220 is compressed by the airway, the two adjacent ablation units 221 have a spacing distance to block or weaken the transmission of force between the two adjacent ablation units 221. When the ablation unit 221 is compressed by the smooth muscle 812, on the one hand, the ablation unit 221 does not interfere with the adjacent ablation units 221 and extends to two ends, thereby making the ablation unit 221 more easily attach to the smooth muscle 812; and on the other hand, the ablation unit 221 does not drive the adjacent ablation units 221 to deform. Thus, the ablation unit 221 on the side away from the smooth muscle 812 is prevented from being pulled and excessively deformed to make it difficult to attach to the C-shaped profile 811, thereby improving the adaptability of the mounting member 220 to the D-shaped profile of the airway.

Further, as shown in Fig. 15, the supporting body 210 includes a plurality of supporting members 211. The plurality of supporting members 211 are provided along the circumference of the mounting member 220 at spaced intervals, and each ablation unit 221 is connected to at least two supporting members 211.

In the embodiment, the supporting body 210 is formed from metal material by laser cutting, and the supporting members 211 are provided along the circumference of the mounting member 220 at equal intervals or at non-equal intervals. The embodiment does not limit the number of the supporting members 211. For example, in one embodiment, the supporting member 211 includes a first supporting arm 2111 and a second supporting arm 2112. One end of the first supporting arm 2111 and one end of the second supporting arm 2112 are connected to form a V-shaped structure, and the other ends of the first supporting arm 2111 and the second supporting arm 2112 are connected to the ablation unit 221. An opening of the V-shaped structure is provided toward the side close to the ablation unit 221. Two ends of each V-shaped structure are connected to two adjacent ablation units 221, respectively, and a plurality of V-shaped structures are provided along the circumference of the mounting member 220.

Thus, the first supporting arm 2111 and the second supporting arm 2112 are connected to form a V-shaped structure. On the one hand, there is a gap between the first supporting arm 2111 and the second supporting arm 2112 to allow a space where two ends of the ablation unit 221 are deformed and elongated so that the ablation unit 221 adhered to the smooth muscle 812 can be compressed and deformed to attach to the smooth muscle 812. On the other hand, when the first region 222 is compressed by the airway and deformed, the transmission of force between two adjacent ablation units 221 is interrupted by the gap between the first supporting arm 2111 and the second supporting arm 2112, thereby increasing the adaptability of the mounting member 220 to the inner wall of the D-shaped profile of the airway.

In another embodiment, the supporting member 211 has a rod-like structure. Each supporting member 211 is connected to only one ablation unit 221, each ablation unit 221 is connected to at least two adjacent supporting members 211, and the two adjacent supporting members 211 are provided at spaced intervals so that a gap may also be formed between the two adjacent supporting members 211, thereby blocking the transmission of force of the two adjacent ablation units 221 and increasing the adaptability of the mounting member 220 to the D-shaped profile of the airway.

Further, the spacing distance between two adjacent ablation units 221 is 0.2-1 mm. Specifically, the spacing distance between two adjacent ablation units 221 can be: 0.2mm, 0.4mm, 0.5mm, 0.8mm, or 1mm. Thus, by limiting the spacing distance of the two adjacent ablation units 221, end portions of the two adjacent ablation units 221 can be attached to form a continuous ablation area in the circumferential direction when the two adjacent ablation units 221 are deformed under pressure.

### Embodiment 3

The embodiment differs from embodiment 1 in that, as shown in Fig. 16, the supporting body 310 includes a plurality of woven wires, and the plurality of woven wires are woven to form a disc-shaped woven body 330. A circumferential edge 320 of the disc-shaped woven body 330 forms a mounting member, and the first region and the second region are located on the circumferential edge 320 of the disc-shaped woven body 330.

In this embodiment, the woven wire of the disc-shaped woven body 330 is a conductive wire, the circumferential edge 320 of the disc-shaped woven body 330 is exposed, and the exposed portion forms a mesh electrode after being energized. In other embodiments, an outer side wall of the disc-shaped woven body 330 is covered with an insulating layer, or the woven wire of the disc-shaped woven body 330 is non-conductive material. The electrode is provided on the outer side wall of the disc-shaped woven body 330 to form a mounting member. At the first region, the woven wires intersect and are fixedly connected, and at the second region, the woven wires intersect and are movably connected. The first region and the second region are provided along the circumference of the disc-shaped woven body 330, and the ratio of lengths of the first region and the second region is similar to that of embodiment 1, which will not be described in detail.

Thus, the disc-shaped woven body 330 is formed by weaving the woven wires through the supporting body 310 and has a good radial supporting force. When the mounting member is compressed by the inner wall of the airway, the supporting body 310 provides a sufficient supporting force to make the mounting member attach to the inner wall of the airway.

Further, the ablation apparatus includes an adjusting member 340. One end of the adjusting member 340 is connected to one axial end of the disc-shaped woven body 330, and the other end of the adjusting member 340 passes through a sleeve 350. The adjusting member 340 is configured to adjust a diameter of the disc-shaped woven body 330.

In this embodiment, two axial ends of the disc-shaped woven body 330 extend to an axially outer side of the disc-shaped woven body 330 to allow a deformation space for diameter adjustment of the disc-shaped woven body 330. One end of the adjusting member 340 is threadably connected or welded to one axial end of the disc-shaped woven body 330, and the other end of the adjusting member 340 passes through the sleeve 350.

In one embodiment, when it is necessary to increase the diameter of the disc-shaped woven body 330, the adjusting member 340 is pulled to move the two axial ends of the disc-shaped woven body 330 towards each other, thereby increasing the diameter of the disc-shaped woven body 330; when it is necessary to increase the diameter of the disc-shaped woven body 330, the adjusting member 340 is pushed to move the two axial ends of the disc-shaped woven body 330 away from each other, thereby reducing the diameter of the disc-shaped woven body 330.

The advantage of this arrangement is that when the supporting body 310 deviates from the target position after being released in the airway, the diameter of the disc-shaped woven body 330 is adjusted by the adjusting member 340 so that the diameter of the disc-shaped woven body 330 is reduced to adjust a position of the disc-shaped woven body 330 in the airway, thereby reducing the difficulty in adjusting the position of the supporting body 310 in the airway.

### Embodiment 4

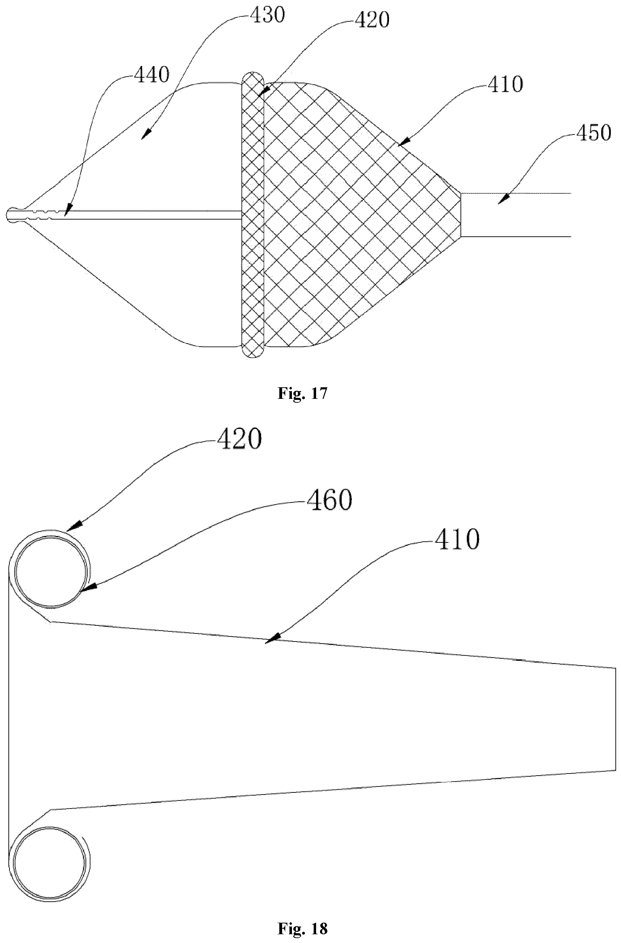

This embodiment differs from embodiment 1 in that, as shown in Fig. 17, the ablation apparatus includes a cooling balloon 430. The cooling balloon 430 is inserted into a supporting body 410, and when the cooling balloon 430 is inflated, it at least partially covers meshes on a mounting member 420.

In this embodiment, the cooling balloon 430 is configured to accommodate a cooling medium. One end of a delivery channel 440 is located in the balloon, and the other end of the delivery channel 440 passes through a sleeve 450. The cooling medium may be inputted into the balloon through a conduit to achieve cooling of the electrode and the site to be ablated. The cooling medium may be a gaseous cooling medium or a liquid cooling medium. Specifically, the cooling medium may be at least one of CO2 gas flow, physiological saline, or purified water.

Side walls of the supporting body 410 and the mounting member 420 are of a mesh structure, and meshes on the mesh structure constitute heat conduction holes. During the process of ablating the airway, the cooling medium is inputted into the cooling balloon 430 to inflate the balloon. After the cooling balloon 430 is inflated, it attaches to the side walls of the supporting body 410 and the mounting member 420 and covers the meshes of the supporting body 410 and the mounting member 420 so that a side wall of the cooling balloon 430 attaches to the inner wall of the airway. The heat on the inner wall of the airway can be transmitted to the cooling balloon 430 through the heat conduction holes, increasing the heat conduction efficiency and improving the cooling effect of the balloon. After the ablation of the airway is completed, the cooling medium in the balloon is withdrawn to deflate the balloon, and the balloon and supporting body 410 are withdrawn.

Thus, when the cooling balloon 430 is inflated, the cooling balloon 430 is inserted into the supporting body 410, and the side wall of the cooling balloon 430 covers the meshes of the supporting body 410 and the mounting member 420 so that the side wall of the cooling balloon 430 attaches to the inner wall of the airway, facilitating the cooling of the electrode and the inner wall of the airway, and avoiding the burn of the inner wall of the airway due to excessive ablation temperature.

In other embodiments, as shown in Fig. 18, the ablation apparatus includes a cooling conduit 460. The cooling conduit 460 is provided around the supporting body 410 and is located on an inner side of the mounting member 420. An outer wall of the cooling conduit 460 attaches to the inner wall of the mounting member 420. Injection holes are arranged on the cooling conduit 460, and positions of the injection holes correspond to positions of the meshes on the mounting member 420. The cooling medium passes through the injection holes and the meshes on the mounting member 420 to be injected to the inner wall of the airway, thereby facilitating the cooling of the inner wall of the airway, and avoiding the burn of the inner wall of the airway due to excessive ablation temperature.

The technical features of the above-mentioned embodiments may be arbitrarily combined, and in order to make the description concise, not all the possible combinations of the technical features in the above-mentioned embodiments are described. However, as long as there is no contradiction between the combinations of these technical features, they should be considered as the scope of the description.

The above-mentioned embodiments express only several embodiments of the present invention, which are described in a specific and detailed manner, but are not to be construed as limiting the patent scope of the present invention. It should be noted that several variations and modifications may be made by a person skilled in the art without departing from the conception of the present invention, all of which fall within the scope of the present invention. Therefore, the scope of the present invention patent shall be subject to the attached claims.

## Claims

1. A nerve ablation apparatus, comprising a supporting body and a mounting member, wherein the mounting member is provided along a circumference of the supporting body and of a mesh structure; the mounting member comprises a first region and a second region provided along the circumference of the supporting body and comprises the mesh structure formed by a plurality of mesh wires; in the first region, the plurality of mesh wires intersect and at least a portion of the mesh wires are fixedly connected at intersections.

2. The nerve ablation apparatus according to claim 1, wherein the mesh wires comprise woven wires, and the mesh structure is formed by weaving the woven wires.

3. The nerve ablation apparatus according to claim 1, wherein an increasing amount of a mesh density in the first region is less than an increasing amount of a mesh density in the second region when the mounting member is compressed towards an inner side of the supporting body.

4. The nerve ablation apparatus according to claim 1, further comprising a buffer structure, wherein the buffer structure is provided at the connected boundary of the first region and the second region and is configured to block the transmission of force between the first region and the second region.

5. The nerve ablation apparatus according to claim 4, wherein the buffer structure comprises a reinforcing member; the reinforcing member is connected to the supporting body and the mesh wires and extends from the supporting body to the connected boundary of the first region and the second region.

6. The nerve ablation apparatus according to claim 1, wherein the mesh wire is made of conductive material to make the mounting member form mesh electrodes.

7. The nerve ablation apparatus according to claim 1, wherein the stiffness of one end of the supporting body close to the mounting member is less than the stiffness of the other end of the supporting body away from the mounting member.

8. The nerve ablation apparatus according to claim 7, wherein the supporting body has a mesh structure, and the area of the smallest mesh of an end portion of the supporting body close to the mounting member is larger than the area of the smallest mesh of an end portion of the supporting body away from the mounting member.

9. The nerve ablation apparatus according to claim 1, wherein the cross-sectional area of the supporting body gradually decreases from one end close to the mounting member to the other end away from the mounting member.

10. The nerve ablation apparatus according to claim 1, wherein at least a portion of the mounting member is bent towards a direction close to the center of the supporting body, and a side wall of the mounting member comprises an arcuate curved surface.

11. The nerve ablation apparatus according to claim 1, wherein the supporting body comprises a disc-shaped woven body formed by weaving at least one woven wire, and the mounting member is arranged on a circumferential side wall of the disc-shaped woven body.

12. The nerve ablation apparatus according to claim 1, further comprising a cooling balloon, wherein the cooling balloon is arranged in the inner side of the supporting body, and after the cooling balloon is inflated, a side wall of the cooling balloon attaches to a side wall of the supporting body.

13. The nerve ablation apparatus according to claim 12, wherein heat conduction holes are arranged on the side wall of the supporting body, and after the cooling balloon is inflated, the cooling balloon covers the heat conduction holes.
